# EUROPEAN PATENT SPECIFICATION

(11) **EP 2 839 830 B1**
(45) Date of publication and mention of the grant of the patent: **15.05.2019**
(21) Application number: 14182345.0
(22) Date of filing: 13.01.2006
(51) Int. Cl.: A61K 31/055, A61K 31/137, A61K 31/167, A61K 31/221, A61K 31/245, A61K 31/40, A61K 31/445, A61K 31/46, A61K 31/47, A61K 31/535, A61K 31/727, A61K 31/728, A61K 31/737, A61K 45/06, A61P 13/02, A61K 9/00, A61K 9/08

(54) **KITS AND COMPOSITIONS FOR TREATING LOWER URINARY TRACT DISORDERS**
KITS UND ZUSAMMENSETZUNGEN ZUR BEHANDLUNG VON ERKRANKUNGEN DER UNTEREN HARNWEGE
KITS ET COMPOSITIONS POUR TRAITER DES TROUBLES DU TRACTUS URINAIRE INFÉRIEUR

(30) Priority: 14.01.2005 US 643885 P; 19.12.2005 US 752287 P
(43) Date of publication of application: 25.02.2015
(62) Divisional of application: 06718460.6
(73) Proprietor: Urigen, Inc., San Francisco CA 94109 (US)
(72) Inventor: PARSONS, C., CA, 94109 San Francisco (US)
(74) Representative: Winter, Brandl, Fürniss, Hübner, Röss, Kaiser, Polte - Partnerschaft mbB

(56) References cited:
- WO-A-2005/072751
- US-A1- 2004 131 590
- PARSONS C L: "Successful downregulation of bladder sensory nerves with combination of heparin and alkalinized lidocaine in patients with interstitial cystitis", UROLOGY, BELLE MEAD, NJ, US, vol. 65, no. 1, 21 January 2005 (2005-01-21), pages 45-48, XP004724790, ISSN: 0090-4295
- PARSONS C LOWELL: "Current strategies for managing interstitial cystitis", EXPERT OPINION ON PHARMACOTHERAPY, ASHLEY, LONDON, vol. 5, no. 2, 1 February 2004 (2004-02-01), pages 287-293, XP008097859, ISSN: 1465-6566
- DELL JEFFREY R ET AL: "Multimodal therapy for interstitial cystitis", JOURNAL OF REPRODUCTIVE MEDICINE, IL, vol. 49, no. 3, Suppl. S, 1 March 2004 (2004-03-01), pages 243-252, XP008097860, ISSN: 0024-7758
- PARSONS C L: "Evidence-based strategies for recognizing and managing IC", CONTEMPORARY UROLOGY, ADVANSTAR COMMUNICATIONS, US, 1 February 2003 (2003-02-01), pages 22-35, XP008097855, ISSN: 1042-2250
- LUKBAN J C: "Current status in the pharmacological management of interstitial cystitis", EXPERT OPINION ON PHARMACOTHERAPY, ASHLEY, LONDON, vol. 4, no. 11, 1 January 2003 (2003-01-01), pages 1967-1975, XP008097868, ISSN: 1465-6566
- LUKBAN J C ET AL: "Interstitial cystitis and pelvic floor dysfunction: A comprehensive review", PAIN MEDICINE 2001 US, vol. 2, no. 1, 2001, pages 60-71, XP002501826, ISSN: 1526-2375
- LUKBAN J C ET AL: "CURRENT MANAGEMENT IN INTERSTITIAL CYSTITIS", UROLOGIC CLINICS OF NORTH AMERICA, SAUNDERS CO., LONDON, GB, vol. 29, no. 3, 1 August 2002 (2002-08-01) , pages 649-660, XP009051896, ISSN: 0094-0143
- WHITMORE K E: "SELF-CARE REGIMENS FOR PATIENTS WITH INTERSTITIAL CYSTITIS", UROLOGIC CLINICS OF NORTH AMERICA, SAUNDERS CO., LONDON, GB, vol. 21, no. 1, 1 February 1994 (1994-02-01), pages 121-130, XP009037042, ISSN: 0094-0143
- DASGUPTA ET AL: "ELECTROMOTIVE DRUG ADMINISTRATION OF LIDOCAINE TO ANESTHETIZE THE BLADDER BEFORE INTRAVESICAL CAPSAICIN", JOURNAL OF UROLOGY, BALTIMORE, MD, US, vol. 159, no. 6, 1 June 1998 (1998-06-01), pages 1857-1861, XP005566059, ISSN: 0022-5347
- HENRY ET AL: "ABSORPTION OF ALKALIZED INTRAVESICAL LIDOCAINE IN NORMAL AND INFLAMED BLADDERS: A SIMPLE METHOD FOR IMPROVING BLADDER ANESTHESIA", JOURNAL OF UROL, LIPPINCOTT WILLIAMS & WILKINS, BALTIMORE, MD, US, vol. 165, no. 6, 1 June 2001 (2001-06-01), pages 1900-1903, XP005544838, ISSN: 0022-5347, DOI: 10.1016/S0022-5347(05)66238-6

## Description

### BACKGROUND OF THE INVENTION

This invention is directed to superior buffered formulations and kits including the superior buffered formulations for treating lower urinary tract symptoms and disorders, as defined in the claims, wherein the pH at which the solution is buffered is from 7.3 to 7.7. In particular superior buffered formulations have demonstrated improvement for treating lower urinary tract symptoms of patients experiencing severe pain and/or urgency of the bladder and associated areas of the lower urinary tract.

A large number of diseases and conditions occur in the lower urinary tract and are associated with one or more pelvic symptoms of pain, urge, frequency, or incontinence. In gynecologic patients, pelvic pain is referred to as chronic pelvic pain and may be of unknown origin or may be related to bacterial cystitis, fungal/yeast cystitis, vulvar vestibulitis, vulvodynia, dysparenunia, endometriosis. Regardless of the perceived source of pelvic pain, in many cases the actual source of pain may be the bladder and/or the lower urinary tract. Frequency and urge together encompass the symptoms of overactive bladder. Overactive bladder may also be associated with incontinence, particularly urge incontinence.

In both male and female patients that are treated with cytotoxic therapies for cancer, this may result in any one or more lower urinary tract symptoms of pelvic pain, urge, frequency or incontinence. Localized radiation therapy to the pelvis which occurs due to bladder, cervical, ovarian, rectum, colon, vagina/vulva or prostate cancer therapy, may result in damaging the epithelium of the bladder wall leading to one or more of lower urinary tract symptoms of pain, urge, and/or frequency. Cytotoxic cancer chemotherapy, most notably cyclophosphamide and ifosfamide treatment for breast cancer patients (male and female) may also lead to the same series of symptoms.

In male patients, any one or more lower urinary tract pelvic symptoms of pelvic pain, urge, frequency or incontinence is observed in patients with prostatitis, chronic pelvic pain syndrome, urethral syndrome, or overactive bladder.

There are no specific treatments for lower urinary tract pelvic pain and instead patients are prescribed oral NSAIDS such as aspirin or acetaminophen. For severe chronic pain, some subjects rely on oral and/or transdermal narcotics which typically results in an irreversible worsening of symptoms.

For the symptoms of urinary urge and frequency, also termed overactive bladder, oral anticholinergic drugs such as detroloxybutynin chloride (Ditropan XL®) and tolterodine (Detrusitol®, Detrol LA®) reduce the contraction of the smooth muscle of the bladder wall. However, these drugs do not treat the underlying cause of the problem. Additionally, these drugs may result in side effects such as dry mouth, constipation, headache, blurred vision, hypertension, drowsiness, and urinary retention in approximately 50%. The benefits of these drugs do not appear to overcome their risks/detriments since only 20% of patients refill their prescriptions.

There is one agent, Mesnex® (mesna) that is used for the prevention of hemorrhagic cystitis due to ifosfamide treatment in cancer patients. This agent is a detoxifying agent and binds and detoxifies the cancer drug. The drug does not treat acute pain and actually results in very high frequency of adverse events (all AEs for IV = 85%, for oral = 89%), most notable adverse events are nausea, vomiting, and constipation.

Although heparinoid-based therapy (heparin; the oral agent pentosan polysulfate sodium [PPS]) is an effective treatment for interstitial cystitis (IC), patients may require several months of therapy or more before they experience relief of pain and urgency/frequency. (P.M. Hanno, "Analysis of Long-Term Elmiron Therapy for Interstitial Cystitis," Urology 49(Suppl 5A): 93-99 (1997)) Heparinoids, which are believed to augment the dysfunctional epithelium that is present in many cases of the disease, take time to reach full effectiveness in reversing the disease process and thereby reducing symptoms. (C. L. Parsons," Epithelial Coating Techniques in the Treatment of Interstitial Cystitis. Urology 49(Suppl 5A):100-104 (1997)). In addition, particularly in severe or long-standing cases of IC, there is significant upregulation of the sensory nerves in the bladder. (T.J. Christmas et al., "Nerve Fibre Proliferation in Interstitial Cystitis," Virchows Archiv. A Pathol. Anat. 416: 447-451 (1990); X. Pang et al., "Increased Number of Substance P Positive Nerve Fibres in Interstitial Cystitis," Br. J. Urol. 75:744-750 (1995); C.A. Buffington & S.A. Wolfe, Jr., "High Affinity Binding Sites for [3H]Substance P in Urinary Bladders of Cats with Interstitial Cystitis," J. Urol. 160:605-611 (1998)). Heparinoids allow natural downregulation of the nerves over time by gradually restoring the barrier function of the mucus and thus preventing further irritation by urinary constituents such as potassium (J.C. Nickel et al., "Randomized, Double-Blind, Dose-Ranging Study of Pentosan Polysulfate Sodium (PPS) for Interstitial Cystitis (IC)," J. Urol. 165(5 Suppl): 67 (2001); C.L. Parsons et al., :"Effect of Pentosan Polysulfate Therapy on Intravesical Potassium Sensitivity," Urology 59: 329-333 (2002)) No currently available IC therapy achieves immediate symptom relief without destroying the nerve endings (T.W. Cannon & M.B. Chancellor, "Pharmacotherapy of the Overactive Bladder and Advances in Drug Delivery," Clin. Obstet. Gynecol. 45: 205-17 (2002); M.B. Chancellor & N. Yoshimura, "Treatment of Interstitial Cystitis," Urology 63(3 Suppl 1): 85-89 (2004); M. Lazzeri et al., "Intravesical Infusion of Resiniferatoxin by a Temporary in Situ Drug Delivery System to Treat Interstitial Cystitis: A Pilot Study," Eur. Urol. 45: 98-102 (2004)) or employing narcotics. Thus there continues to be a need for an IC treatment that offers immediate relief of symptoms and operates directly to downregulate the bladder sensory nerves without any rebound effect.

Intravesical agents have been used for many years as adjuncts to oral treatment regimens or as second-line therapies for IC. One of the most widely used is heparin, which is effective in approximately 50% of patients treated. Heparin is a sulfated polysaccharide that is believed to augment the protective effect of the natural bladder surface mucus. Intravesical heparinoid agents alone, however, do not produce immediate and sustained relief of IC symptoms. Like the oral heparinoids, they take several months to produce symptom relief.

Other treatments have also been tried, with limited success. For example, treatments with dimethylsulfoxide (DMSO), approved for IC in 1977 on the basis of data from uncontrolled trials, can be useful with weekly intravesical instillations for 6 to 8 weeks then every two weeks for 3-12 months for maintenance. However DMSO therapy results in benefit for approximately only 50% of IC patients treated and the treatment takes a long time to reduce symptoms. Furthermore, this therapy causes pain that is unrelieved by local anesthetics by themselves due to their lack of absorption into the bladder wall. Narcotics are given for immediate relief of symptoms however they are only minimally effective. The use of narcotics, of course, carries a significant risk of tolerance and addiction. Some patients benefit from formal 8- to 12-week, one-on-one course of behavior modification. Patients are also advised to avoid potassium-rich foods, particularly citrus fruits, tomatoes, chocolate, and coffee.

Many urologists treat interstitial cystitis patients with their own "home-brew" of drugs by administering the drug(s) or mixtures thereof into the lumen of the bladder. As these procedures are typically done in the office without any quantitative assessment of severity of initial symptoms prior to or subsequent to treatment, there is no scientific rigor in assessing the benefit of these treatments. Consequently, patients are treated with drugs in their non-approved indications with no real scientific guidance as to whether the patient will benefit from the treatment or not.

Consequently, there is a tremendous need for scientifically-validated and improved treatments that provide immediate relief for treating lower urinary tract symptoms and disorders, particularly those with severe interstitial cystitis. Additionally, these treatments should be based on validated quantitative assessment of benefit, not on wishful thinking which has been the basis of urologists "home-brew" treatments that are not assessed quantitatively. There is a particular need for improved treatments and compositions for use in those treatments that provide immediate relief and do not require several months until the patients experience relief.

Previously, Parsons (Parsons, CL "Evidence-based strategies for recognizing and managing IC" Contemporary Urology, February, pps. 22-35, 2003) published a recipe of three FDA-approved drug components for the treatment of interstitial cystitis, which is a painful bladder disorder of unknown etiology. The components were 80 mg lidocaine (8 ml 1% lidocaine), 40,000 units heparin (4 ml of 10,000 units/ml heparin sodium), and 252 mg bicarbonate (3ml of 8.4% sodium bicarbonate) in a total aqueous volume of 15ml.

WO 2005/072751 A discloses a composition comprising a heparinoid, a local anesthetic agent, and buffering compound for the treatment of lower urinary tract disorder.

Already known is a treatment of the lower urinary tract disorder interstitial cystitis (IC) (Parsons, C L "Current strategies for managing interstitial cystitis" Expert Opinion of Pharmacotherapy, 5(2), 287-293, 2004; Dell J R et al. "Multimodal therapy for interstitial cystitis" Journal of Reproductive Medicine, 49(3), 243-252, 2004), comprising instillation of a solution containing 10000-40000 U of heparin dissolved in 10 ml buffered sodium chloride, 10 ml 1% lidocaine or 16 ml 2% lidocaine and 3 ml 8,4% sodium bicarbonate.

An additional limitation of the Parsons approach is that components have to be measured out immediately before use from three separate solutions. In many treatment settings such as clinics or doctor's offices there are neither the pharmaceutical personnel resources qualified to measure out these components from stock solutions or the possibility exists of accidental mis-measurement leading to the potential for incorrect treatment or lidocaine overdose. Additionally, this mixing in a non-sterile environment may result in contamination with an infectious agent or other detrimental component that would be directly instilled in a compromised bladder. Consequently, this invention provides for pre-measured kits and prefilled vials of the formulation of the invention that prevents these point-of-care problems.

An aspect of the previous formulation by Parsons is that it contained multiple components and with any solution or equation with multiple variables, it cannot be clear what portion of the each component contributes to the effect. Accordingly, there is a need for an improvement in the formulation that would maximize the effect of each component.

### SUMMARY OF THE INVENTION

The present invention embodies improved compositions according to claim 1 and multipart kits according to claim 9 for treating pelvic disorders. The invention is defined by the claims. Any subject matter falling outside the scope of the claims is provided for information purposes only.

### BRIEF DESCRIPTION OF THE DRAWINGS

The following invention will become better understood with reference to the specification, appended claims, and accompanying drawings, where:

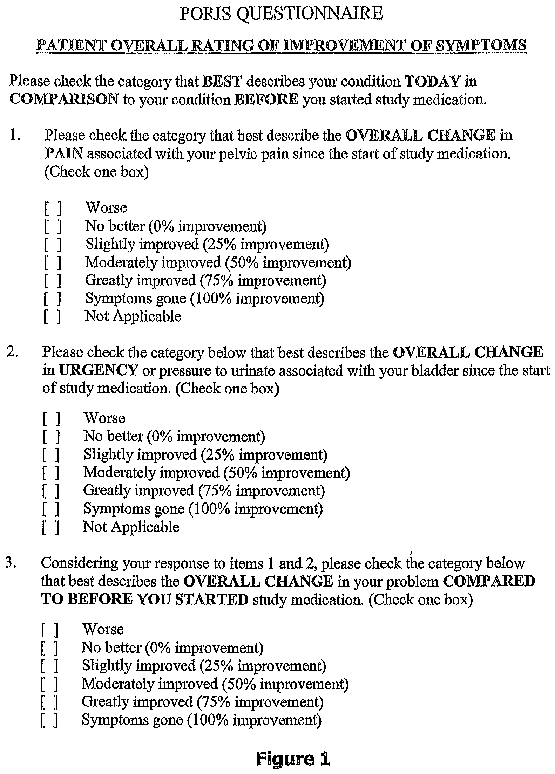

Figure 1 is the validated PORIS questionnaire, "Patient Overall Improvement of Symptoms" that has been used for the approval of oral Elmiron® (pentosan polysulfate sodium) with the U.S. FDA. The primary endpoint for that approval and for the study in the current invention was "Moderately improved"; i.e., 50% or greater improvement in overall change in combined symptoms of pain and/or urgency since the start of therapy.

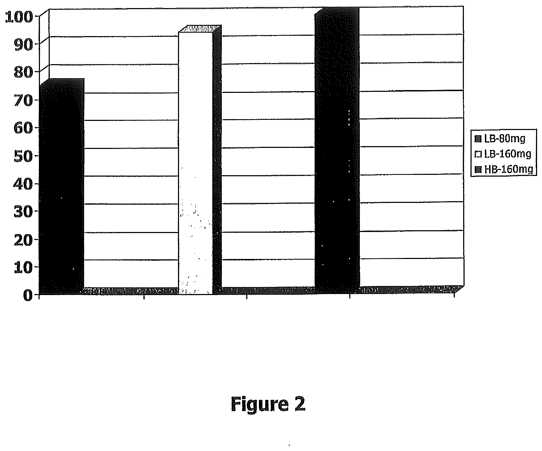

Figure 2 is a graph comparing the PORIS results on a preferred composition according to the present disclosure with 160 mg of lidocaine per unit dose and 0.33 M sodium bicarbonate as buffer ("HB-160 mg"), a superior buffered formulation, versus a low buffer formulation (sodium bicarbonate at 0.2 M) with 160 mg lidocaine ("LB-160 mg") and low buffer with 80 mg lidocaine ("LB-80 mg"). 26 patients were treated with HB-160 mg, 35 were treated with LB-160 mg and 47 were treated with LB-80 mg. A positive result was if a patient exhibited "moderately improved" combined symptoms of pain and urgency - PORIS question 3. Furthermore, 6 patients were on chronic narcotic use for greater than 6 months, and 4/6 of these patients experienced 100% relief on PORIS scale and the remaining 2/6 experienced 75% relief on PORIS scale. Additionally, the patients treated with HB-160 mg had fairly severe symptoms of pain and/or urge with 15/26 (58%) had PUF scores >20. All patients had greater than 15 PUF score.

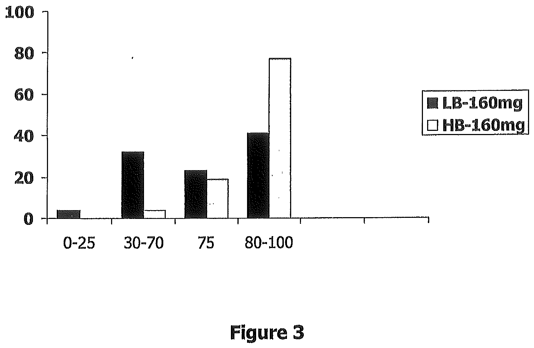

Figure 3 is a graph comparing the relative distribution of PORIS scores between patients administered the study drug of the current invention, HB-160 mg versus LB-160 mg. More patients experienced a 100% improvement in symptoms (>75% of patients) versus the older formulation, LB-160 mg in which only about 40% of patients experienced a 100% improvement in combined symptoms of pain and urgency. Furthermore, with the HB-160 mg formulation patients had >25% improvement in symptoms of pain and urgency whereas with the LB-160 mg, a small group of patients was essentially unresponsive (0-25% improvement in symptoms). Consequently, validated PORIS patient responses clearly demonstrate superiority of the formulation of the present invention over LB-160 mg with a statistically significant P value of 0.009.

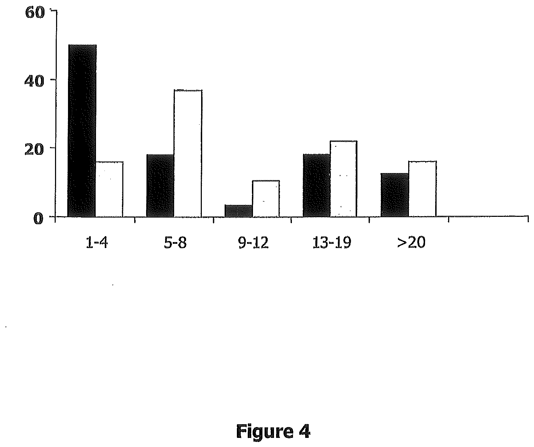

Figure 4 is a graph of the duration of relief of combined symptoms of pain and urgency of the formulation according to the present invention, HB-160 mg. Study patients were followed up 24 hr after treatment in the clinic with study drug and asked how long of a benefit from the treatment they received. The half-life of lidocaine when used for its anesthetic effect alone is 1.5 hr. For the patients treated with the HB-160 mg formulation, the distribution of time was much longer and median duration was 7 hours, significantly longer than predicted than the half-life of lidocaine. In comparison, the median duration of benefit of LB-160 mg was only 4 hours.

### DETAILED DESCRIPTION OF THE INVENTION

One aspect of the invention is a pharmaceutical composition for treating or ameliorating a lower urinary tract disorder. In general, an improved composition according to the present invention has as its basis a composition comprising:
(1) an anionic polysaccharide in a quantity sufficient to treat or ameliorate the lower urinary tract disorder;
(2) lidocaine in a quantity sufficient to treat or ameliorate the lower urinary tract disorder;
(3) a buffer that buffers the solution at a pH that ensures that a sufficient portion of the acute-acting anesthetic is present in an uncharged state so that the acute-acting anesthetic can cross the cell membranes; and
(4) optionally, an osmolar component that provides an isotonic or nearly isotonic solution compatible with human cells and blood.

The lower urinary tract disorder treatable by the use of compositions according to the present invention is interstitial cystitis (IC), which is more frequently diagnosed in women, but is now being diagnosed more frequently in men as well. Another lower urinary tract disorder treatable by the use of compositions according to the present invention is radiation-induced cystitis.

Typically, the quantity of lidocaine in the composition is such that a unit dose contains from 120 mg to the maximum safely tolerated dose of lidocaine. Preferably, the quantity of lidocaine in the composition is such that a unit dose contains 160 mg to 240 mg of lidocaine. More preferably, the quantity of lidocaine in the composition is such that a unit dose contains 160 mg of lidocaine. Alternatively, the quantity of lidocaine in the composition is such that a unit dose contains 200 mg of lidocaine or 240 mg of lidocaine.

However, when the composition includes one or more of: (1) a compound that enables persistence of the composition to the surface of the bladder epithelium in a quantity sufficient to treat or ameliorate the lower urinary tract disorder; (2) an antibacterial agent in a quantity sufficient to treat or ameliorate the lower urinary tract disorder; (3) an antifungal agent in a quantity sufficient to treat or ameliorate the lower urinary tract disorder; or (4) a vasoconstrictor in a quantity sufficient to treat or ameliorate the lower urinary tract disorder, as described below, and the acute-acting anesthetic is lidocaine, typically, the quantity of lidocaine is such that a unit dose contains from 80 mg to the maximum safely tolerated dose of lidocaine. In this alternative, when one or more additional components are included, the quantity of lidocaine per unit dose can be 80 mg, 120 mg, 160 mg, 200 mg, or 240 mg.

The buffer is present in a quantity such that the buffer has a buffering capacity at least equivalent to the buffering capacity of a quantity of sodium bicarbonate such that, when the formulation is dissolved in an aqueous liquid for administration, the sodium bicarbonate is present at a concentration of 0.20 M to 0.45 M. Preferably, the buffer is present in a quantity such that the buffer has a buffering capacity equivalent to the buffering capacity of a quantity of sodium bicarbonate such that, when the formulation is dissolved in an aqueous liquid for administration, the sodium bicarbonate is present at a concentration of 0.33 M.

Typically, the buffer is sodium bicarbonate. The sodium bicarbonate is present in a quantity such that when the formulation is dissolved in an aqueous liquid for administration, the sodium bicarbonate is present at a concentration of from 0.20 M to 0.45 M. Preferably, the sodium bicarbonate is present in a quantity such that when the formulation is dissolved in an aqueous liquid for administration, the sodium bicarbonate is present at a concentration of from 0.30 M to 0.45 M. More preferably, the sodium bicarbonate is present in a quantity such that when the formulation is dissolved in an aqueous liquid for administration, the sodium bicarbonate is present at a concentration of 0.33 M. The pH of the composition is from 7.3 to 7.7. More preferably, the pH of the composition is from 7.4 to 7.5.

Typically, when the anionic polysaccharide is heparin, the heparin is present at a concentration of from 2500 U/ml to 4166 U/ml. Preferably, when the anionic polysaccharide is heparin, the heparin is present at a concentration of from 3000 U/ml to 3666 U/ml. More preferably, when the anionic polysaccharide is heparin, the heparin is present at a concentration of 3333 U/ml. These concentrations are determined based on a volume of 12 ml per unit dose and can be adjusted if the volume of the unit dose is changed.

Typically, lidocaine is present at a concentration of from 34.5 mM to 64 mM. Preferably, the lidocaine is present at a concentration of from 41.4 mM to 50.6 mM. More preferably, the lidocaine is present at a concentration of 46 mM. These concentrations are determined based on a volume of 12 ml per unit dose and can be adjusted if the volume of the unit dose is changed.

Typically, when the osmolar component is sodium chloride, the sodium chloride is present at a concentration of from 21.4 mM to 150.0 mM. Preferably, when the osmolar component is sodium chloride, the sodium chloride is present at a concentration of from 25.6 mM to 31.4 mM. More preferably, when the osmolar component is sodium chloride, the sodium chloride is present at a concentration of 28.5 mM. These concentrations are determined based on a volume of 12 ml per unit dose and can be adjusted if the volume of the unit dose is changed.

For a composition according to the present disclosure, comprising lidocaine, heparin, sodium bicarbonate, and sodium chloride, amounts and concentrations, as well as the resulting molarities where appropriate, are shown below in Table 1.

**TABLE 1**

| COMPONENTS OF PREFERRED COMPOSITION | | | |
|---|---|---|---|
| **Component** | **Amount** | **Conc.** | **Molarity** |
| **Lidocaine** | 160 mg | 1.33% | 46 mM |
| **Heparin** | 40,000 U | 3,333 U/ml | |
| **Sodium Bicarbonate** | 336 mg | 2.8% | 0.33 M |
| **Sodium Chloride** | 20 mg | 0.2% | 28.5 mM |
| **Total Volume** | 12 ml | | |

For another composition according to the present disclosure, comprising lidocaine, heparin, sodium bicarbonate, and sodium chloride, amounts and concentrations, as well as the resulting molarities where appropriate, are shown below in Table 2.

**TABLE 2**

| COMPONENTS OF PREFERRED COMPOSITION | | | |
|---|---|---|---|
| **Component** | **Amount** | **Conc.** | **Molarity** |
| **Lidocaine** | 200 mg | 1.53% | 53 mM |
| **Heparin** | 40,000 U | 3,077 U/ml | |
| **Sodium Bicarbonate** | 336 mg | 2.58% | 0.305 M |
| **Sodium Chloride** | 20 mg | 0.185% | 26.3 mM |
| **Total Volume** | 13 ml | | |

For yet another composition according to the present disclosure, comprising lidocaine, heparin, sodium bicarbonate, and sodium chloride, particularly preferred amounts and concentrations, as well as the resulting molarities where appropriate, are shown below in Table 3.

**TABLE 3**

| COMPONENTS OF PREFERRED COMPOSITION | | | |
|---|---|---|---|
| **Component** | **Amount** | **Conc.** | **Molarity** |
| **Lidocaine** | 240 mg | 1.71% | 64 mM |
| **Heparin** | 40,000 U | 2,857 U/ml | |
| **Sodium Bicarbonate** | 336 mg | 2.4% | 0.28 M |
| **Sodium Chloride** | 20 mg | 0.17% | 24.4 mM |
| **Total Volume** | 14 ml | | |

The lower urinary tract disorder treatable by the use of compositions according to the present invention is interstitial cystitis (IC), which is more frequently diagnosed in women, but is now being diagnosed more frequently in men as well. Another lower urinary tract disorder treatable by the use of compositions and methods according to the present invention is radiation-induced cystitis.

The anionic polysaccharide is typically a glycosaminoglycan. Glycosaminoglycans are abundant naturally occurring polysaccharides that have a net negative charge due to carboxylic acid or sulfate groups or both. Although Applicants are not bound by this theory, these polysaccharides are believed to have protective effects on the epithelium and to counteract the abnormal permeability of the epithelium to potassium that is characteristic of this condition. Anionic polysaccharides include hyaluronic acid, hyaluronan, chondroitin sulfate, pentosan polysulfate, dermatan sulfates, heparin, heparan sulfates, and keratan sulfates. Heparin exists in a variety of forms characterized by different degrees of sulfation. Typically, heparin has a molecular weight of from 2 kDa to 40 kDa. Heparin and heparan sulfate are both characterized by repeating units of disaccharides containing a uronic acid (glucuronic acid or iduronic acid) and glucosamine, which is either N-sulfated or N-acetylated. The sugar residues can be further O-sulfated at the C-6 and C-3 positions and the C-2 position of the uronic acid. There are at least 32 potential unique disaccharide units in this class of compounds. Five examples of sugars occurring in heparin are: (1) α-L-iduronic acid 2-sulfate; (2) 2-deoxy-2-sulfamino-α-D-glucose 6-sulfate; (3) β-D-glucuronic acid, (4) 2-acetamido-2-deoxy-α-D-glucose, and (5) α-L-iduronic acid. Heparin is measured by its specific anticoagulation activity in units. As used herein, the term "units" refers to specific activity in International Units (IU) and/or United States Pharmacopoeia (USP) units. As used herein, the term "USP unit" refers to the quantity of heparin that prevents 1.0 ml of citrated sheep plasma from clotting for 1 hour after the addition of 0.2 ml of 1% CaCl₂ at 20°C when compared to a USP reference standard (defined as units/ml). As used herein, the term "International Unit" or "IU" refers to the quantity of heparin that is active in assays as established by the Fifth International standard for Unfractionated Heparin (WHO-5) (defined as International Units/ml) (Linhardt, R. J. & Gunay, N. S. (1999) Semin Thromb Hemost 25, 5-16.). In some embodiments, heparin is a higher molecular weight species ranging from 8,000 to 40,000 daltons. As used herein," low-molecular-weight heparins" refers to a lower molecular weight (LMW) species ranging from 2,000 to 8,000 daltons. Also included as glycosaminoglycans within the scope of the invention are pentosan polysulfate sodium of molecular weight ranging from 4,000 to 6,000 daltons, dalteparin, enoxaparin and the like. LMW heparins are made by enzymatic or chemical controlled hydrolysis of unfractionated heparin and have very similar chemical structure to unfractionated heparin except for some changes that may have been introduced due to the enzymatic or chemical treatment. While not intending to limit the mechanism of action of the invention's compositions, it is the inventors' view that mechanism of action of these drugs is similar to that of full-length heparin. LMW heparins are usually isolated from bulk heparin. Because of the negative charge of these polysaccharides due to the occurrence of sulfate groups and/or carboxylic acid groups in them, they are administered in the form of salts, with an appropriate cation to neutralize the negative charges on the acid groups. Typically, the cation is sodium. However, other physiologically tolerable counterions that do not induce urinary tract dysfunctions such as magnesium and aluminum, as well as salts made from physiologically acceptable organic bases such as, but not limited to, trimethylamine, triethylamine, morpholine, pyridine, piperidine, picoline, dicyclohexylamine, N,N'-dibenzylethylenediamine, 2-hydroxyethylamine, bis-(2-hydroxyethyl)amine, tri-(2-hydroxyethyl)amine, dibenzylpiperidine, N-benzyl-β-phenethylamine, dehydroabietylamine, N,N'-bisdehydroabietylamine, glucamine, N-methylglucamine, collidine, quinine, quinoline, and basic amino acids such as lysine and arginine, can be used. These cationic counterions can alternatively be used as the counterions with anionic buffers such as bicarbonate, as well. These salts may be prepared by methods known to those skilled in the art. However, it is generally undesirable to use potassium as a counterion due to its role in the etiology of the conditions and syndromes being treated.

Typically, if heparin is administered, the quantity of heparin is from 10,000 units to 100,000 units per dose in the composition. Preferably, the quantity of heparin in the composition is from 25,000 units to 60,000 units per dose. More preferably, the quantity of heparin in the composition is 40,000 units per dose. Heparin is typically quantitated by its activity; for purified heparin, there are approximately 170 units/mg. Therefore, the preferable dose of 40,000 units is 235.3 mg of heparin.

The quantities for other polysaccharides can be chosen according to their activity by one of ordinary skill in the art, as described further below.

As used herein, the recitation of lidocaine includes all salts of that anesthetic that are compatible with the desired pH and any counterions present; the recitation of lidocaine is not intended to limit the salt form or counterion used.

The effect of the use of lidocaine is not only to provide topical anesthesia. The use of lidocaine is believed to desensitize the nerves involved past the duration of their anesthetic activity. The desensitization may reverse the hypersensitization that occurs in the condition being treated. The hypersensitization may be due to peripheral nerve stimulation, inflammation and/or injury from potassium, nerve regeneration and growth of new nerve fibers, and/or central activation of the sacral reflex arc. In addition, this cascade may be promoted by interactions with mast cells.

For lidocaine to function properly, it typically needs to be in a pH environment that enables the chemical form of the drug to be neutral thereby allowing the molecule to traverse the membranes and gain access to the neurons underlying the bladder epithelium. To achieve this pH, a buffer must be provided so that the pH is sufficiently near to the isoelectric point of the chosen anesthetic to ensure that a sufficient portion of lidocaine is present in an uncharged state. A sufficient portion can minimally be 20% up to 100% in its uncharged state and those skilled in the art can use the drug's isoelectric point and the Henderson-Hasselbalch equation to calculate the minimal pH of the solution to achieve the 20% minimal uncharged amount. Preferably, the pH of the solution is such that at least 40% of lidocaine is present in an uncharged state. This buffering is achieved by the addition of bicarbonate buffer, Tris (Tris(hydroxymethyl)aminomethane) buffer, phosphate buffer. The buffer to be selected, and the concentration of the buffer to be used, can be chosen by one of ordinary skill in the art to buffer the composition to be administered at a pH value that is close to the isoelectric point of the local anesthetic. The pH achieved by the use of the buffer is between 7.3 and 7.7. Typically, when bicarbonate buffer is used, it is sodium bicarbonate buffer.

Due to the inflamed, permeable nature of the urothelium, a preferred solution would be isotonic or near isotonic. Hypotonic solutions are known to result in cell lysis, particularly of red blood cells, but other cells may also be damaged leading to increased cell damage in the bladder and accessible underlying layers. Hypertonic solutions may result in cell shrinkage which may enlarge pores or other junctions allowing urinary solutes more access to underlying cell layers leading to further damage, pain and inflammation. The addition of an osmolar component to the composition to form an isotonic or near isotonic solution ensures that neither of these two possibilities occurs. Typically, the osmolar component is 0.9% sodium chloride, or somewhat less as the other components in the solution also contribute to the solution's osmolarity and thus should be taken into account. Typically the osmolar component is a salt, such as sodium chloride, or a sugar or a combination of two or more of these components. The sugar may be a monosaccharide such as dextrose, a disaccharide such as sucrose or lactose, a polysaccharide such as dextran 40, dextran 60, or starch, or a sugar alcohol such as mannitol. It should be obvious to those skilled in the art that all components of the composition contribute to the osmolarity of the solution but to achieve an isotonic or near-isotonic solution, the contributions of these components should be taken into account to ensure that the proper osmolar component is added and not added in excess which would result in a hypertonic solution.

As indicated above, compositions according to the present invention can include other ingredients. It is possible to include a compound that enables persistence of the composition to the surface of the bladder wall in the composition. A suitable compound that enables persistence of the composition to the surface of the bladder wall is an activatable gelling agent. The addition of an activatable gelling agent that would result in the formation of a gel on the bladder epithelial surface would ensure improved transference of the active drugs (the anesthetic and the bladder coating anionic polysaccharide) to the areas most needing them. In one instance the gelling agent is liquid at room temperature and then upon bladder instillation, would gel at body temperature; in other words, the activatable gelling agent is a thermoreversible gelling agent. This feature of thermoreversible gelation has been observed for Pluronics F127 gel, Lutrol gel (T. Beynon et al., "Lutrol Gel: A Potential Role in Wounds?," J. Pain Symptom Manage. 26: 776-780 (2003)), NASI-containing polymers (N-isopropylacrylamide, ethylmethacrylate, N-acryloxysuccinimide) (T.J. Gao et al., "Synthetic Thermoreversible Polymers Are Compatible with Osteoinductive Activity of Recombinant Human Bone Morphogenetic Protein 2," Tissue Eng. 8: 429-440 (2002); T. Gao & U.H. Uludag, "Effect of Molecular Weight of Thermoreversible Polymer on in Vivo Retention of rhBMP-2," J. Biomed. Mater. Res. 57: 92-100 (2001)), xyloglucan sols of 1-2% (S. Miyazaki et al., "Thermally Reversible Xyloglucan Gels as Vehicles for Rectal Drug Delivery," J. Control Release 4: 75-83 (1998)), graft copolymers of pluronic and poly(acrylic acid), pluronic-chitosan hydrogels, and a [Poly(ethylene glycol)-Poly[lactic acid-co-glycolic acid]-Poly(ethylene glycol)) (PEG-PLGA-PEG) polymer (P. Tyagi et al., "Sustained Intravesical Drug Delivery Using Thermosensitive Hydrogel," Pharm. Res. 21: 832-837 (2004)). In general, these polymers are sols at room temperature but form gels at body temperature, about 37°C. Other activatable gelling agents are known in the art.

In yet another alternative, the composition can further comprise an antibacterial agent or an antifungal agent to treat bacterial or fungal cystitis. Suitable antibacterial agents include, but are not limited to: (1) sulfonamides such as sulfanilamide, sulfadiazine, sulfamethoxazole, sulfisoxazole, sulfamethizole, ; sulfadoxine, and sulfacetamide; (2) penicillins such as methicillin, nafcillin, oxacillin, cloxacillin, dicloxacillin, ampicillin, amoxicillin, bacampicillin, carbenicillin, ticarcillin, mezlocillin, and piperacillin; (3) a combination of trimethoprim plus sulfamethoxazole; (4) quinolones such as nalidixic acid, cinoxacin, norfloxacin, ciprofloxacin, orfloxacin, sparfloxacin, lomefloxacin, fleroxacin, pefloxacin, and amifloxacin; (5) methenamine; (6) nitrofurantoin; (7) cephalosporins such as cephalothin, cephazolin, cephalexin, cefadroxil, cefamandole, cefoxatin, cefaclor, cefuroxime, loracarbef, cefonicid, cefotetan; ceforanide, cefotaxime, cefpodoxime proxetil, ceftizoxime, ceftriaxone, cefoperazone, ceftazidime, and cefepime; (8) carbapenems such as imipenem, meropenem, and aztreonam; (9) aminoglycosides such as netilmycin and gentamicin; (10) tetracyclines such as tetracycline, oxytetracycline, demeclocycline, minocycline, doxycycline, and chlortetracycline; and (11) macrolides such as erythromycin, clarithromycin, and azithromycin. Antifungal agents include amphotericin B, itraconazole, ketoconazole, fluconazole, miconazole, and flucytosine. Other suitable antibacterial agents and antifungal agents are known in the art.

In yet another alternative, the compositions according to the present invention can include a vasoconstrictor. The purpose of a vasoconstrictor is to constrict the blood vessels locally at or near the site of administration to ensure that the composition has its maximum effect at or near the site of administration. A particularly preferred vasoconstrictor is epinephrine. Typically, if the anesthetic is lidocaine, the ratio of epinephrine to lidocaine is from 1:1000 to 1: 200,000. Preferably, if the anesthetic is lidocaine, the ratio of epinephrine to lidocaine is 1:100,000.

Preferred amounts and concentrations are as described above. Particularly preferred amounts and concentrations are described in Table 1, Table 2, and Table 3.

Accordingly, a composition according to the present disclosure comprises:
(1) 160 mg lidocaine per unit dose;
(2) 40,000 units of heparin per unit dose;
(3) 336 mg sodium bicarbonate per unit dose; and
(4) 20 mg sodium chloride per unit dose;
such that, in a final dose volume of 12 ml, lidocaine is present at 46 mM, heparin is present at 3333 units/ml, sodium bicarbonate is present at 0.33 M, and sodium chloride is present at 28.5 mM.

Accordingly, another composition according to the present disclosure comprises:
(1) 200 mg lidocaine per unit dose;
(2) 40,000 units of heparin per unit dose;
(3) 336 mg sodium bicarbonate per unit dose; and
(4) 20 mg sodium chloride per unit dose;
such that, in a final dose volume of 13 ml, lidocaine is present at 53 mM, heparin is present at 3077 units/ml, sodium bicarbonate is present at 0.305 M, and sodium chloride is present at 26.3 mM.

Accordingly, yet another composition according to the present disclosure comprises:
(1) 240 mg lidocaine per unit dose;
(2) 40,000 units of heparin per unit dose;
(3) 336 mg sodium bicarbonate per unit dose; and
(4) 20 mg sodium chloride per unit dose;
such that, in a final dose volume of 14 ml, lidocaine is present at 64 mM, heparin is present at 2857 units/ml, sodium bicarbonate is present at 0.28 M, and sodium chloride is present at 24.4 mM.

Compositions according to the present invention are instilled into the bladder, a route of administration that is referred to as intravesical administration. Typically, this is performed by catheterization. Suitable catheters, and methods for installing the catheters, delivering the composition, and removing the catheters are known in the art and need not be described further here. Typical catheters are made of elastic, elastic web, rubber, glass, metal, or plastic.

Typically, compositions according to the present invention are administered 3-7 times weekly for three weeks or more or on an "as-needed" basis to control acute symptoms of pain and urgency of the lower urinary tract. The treating physician can adjust the frequency and duration of treatment according to the response of the patient, the severity of the symptoms, the degree of pain and discomfort subjectively experienced by the patient, and other factors such as the results of histological tests.

Additionally, still other ingredients can be included in compositions according to the present invention. Such ingredients can include, for example, a coloring agent, a preservative, an antioxidant, a chelating agent, and other ingredients typically used in pharmaceutical formulations. For example, a non-toxic, non-allergenic, non-sensitizing coloring agent can be added for convenience in dispensing and administering the composition. Such coloring agents are well known in the art and are used in many liquid pharmaceutical compositions. Suitable examples of preservatives include, for example, parabens, chlorobutanol, phenol, sorbic acid, or thimerosal.

If sterilization of the composition is required, it is typically performed by filtration. Other sterilization methods are known in the art.

The exact formulation and dosage can be chosen by the individual physician in view of the patient's condition. (See e.g. Fingl et al., in The Pharmacological Basis of Therapeutics, 1975, Ch. 1 p. 1). It should be noted that the attending physician would know how to and when to terminate, interrupt, or adjust administration due to toxicity, or to organ dysfunctions. Conversely, the attending physician would also know to adjust treatment to higher levels if the clinical response were not adequate (precluding toxicity). The magnitude of an administered dose in the management of the disorder of interest will vary with the severity of the condition to be treated, the general condition of the urinary tract, including the bladder and urethra, and the existence of other conditions affecting the urinary tract, such as infections, inflammation, or allergic reactions. The severity of the condition may, for example, be evaluated, in part, by standard prognostic evaluation methods, including the ones described below. Further, the dose, and perhaps the dose frequency, will also vary according to the age, body weight, and response of the individual patient. A program comparable to that discussed above may be used in veterinary medicine.

One prognostic evaluation method is the Pelvic Pain and Urgency/Frequency Patient Symptom Scale (PUF Scale) (Parsons, et al. Urology 60:573-578 (2002)). The PUF Scale is a self-administered questionnaire that can be completed by the patient in less than 5 minutes. It contains questions that elicit and quantify urinary frequency and/or urgency (if any), pelvic pain, and/or pain associated with sexual activity. The result is a single numeric score from 0 through 35. The higher the PUF score, the greater the likelihood that the individual has interstitial cystitis, one of the conditions referred to above (Parsons, et al. Urology 60:573-578 (2002)); for this reason, the PUF can be useful in distinguishing interstitial cystitis from other disorders during the process of diagnosis.

Another prognostic test that is useful is the Potassium Sensitivity Test (PST). Bladder epithelial permeability and urinary potassium appear to play a key role in the development of many cases of the disease [Parsons, et al. J Urol 159:1862-1867 (1998)]. In the healthy bladder, a mucus layer containing glycosaminoglycans (GAGs) forms a barrier that prevents urine and its contents from leaking through the urothelium and damaging the underlying nerves and muscle [Lilly and Parsons, Surg Gynecol Obstet 171:493-496 (1990)]. Most individuals with IC have an epithelial dysfunction that renders the urothelium abnormally permeable. As a result, potentially harmful substances in urine are allowed to leak through the epithelium and penetrate the bladder muscle. Potassium, which occurs in high concentrations in normal urine, does not damage or penetrate a healthy urothelium but is highly toxic to tissues such as the bladder musculature. The depolarization of sensory nerves in the bladder muscle by potassium could produce the symptoms of IC as well as cause its progression. A growing body of data supports this hypothesis. On the basis of this model of IC pathogenesis, the Potassium Sensitivity Test (PST) was developed to test for the presence of abnormal bladder epithelial permeability. The use of the PST is described, for example in Parsons, et al. Urology 57:428-33 (2001), Parsons and Albo, J Urol 168:1054-1057 (2002); Koziol, Urol Clin North Am 21:7-71 (1994). The PST has been positive in 78% of those IC patients tested, providing considerable evidence most IC patients have a urothelial permeability defect, and that a positive PST is a valid indicator of the presence of IC [Parsons, et al. Urology 57:428-33 (2001); Parsons and Albo, J Urol 168:1054-1057 (2002).

Other prognostic tests can be used to determine the existence and severity of the conditions described above. These tests are known to clinicians and others of ordinary skill in the art.

Another aspect of the composition is a multipart kit of two or more separate premeasured components comprising:
(1) a first component that comprises a pharmaceutical composition comprising a solution or dry powder comprising an anionic polysaccharide in a quantity sufficient to treat or ameliorate a lower urinary tract disorder as defined in claim 9;
(2) a second component that comprises lidocaine as a solution or dry powder in a quantity sufficient to treat or ameliorate a lower urinary tract disorder; and
(3) a third component that comprises a buffer either as a solution or as a dry powder that buffers the solution at a pH that ensures that a sufficient portion of the acute-acting anesthetic is present in an uncharged state so that the acute-acting anesthetic can cross the cell membranes, wherein the third component can optionally be combined with the second component as either a solution or dry powder, and wherein the buffer has a buffering capacity at least equivalent to the buffering capacity of a quantity of sodium bicarbonate such that, when the elements comprising the multipart kit are dissolved in an aqueous liquid for administration, the sodium bicarbonate is present at a concentration of from 0.20 M to 0.45 M and wherein the pH at which the solution is buffered is from 7.3 to 7.7 and;
(4) optionally, when the first, second, and third components are all dry powder, a fourth component that comprises a premeasured component of liquid diluent;
(5) optionally, a fifth premeasured component comprising an osmolar component as defined in claim 9; wherein the fifth premeasured component can optionally be combined with any of the other components if stable; and
(6) optionally, a sixth premeasured component comprising one or more of the following in any combination:
   (a) a compound that enables persistence of the composition to the surface of the bladder epithelium as defined in claim 9;
   (b) an antibacterial agent;
   (c) an antifungal agent; and
   (d) a vasoconstrictor; wherein the sixth premeasured component can optionally be combined with any of the other components if stable; wherein the multipart kit is combined to produce a composition that is an intravesical formulation for bladder instillation.

The third component may also be combined with the second component as either a solution or dry powder. If all components are dry powder, then an additional premeasured component of liquid diluent (e.g. sterile water) will be provided for immediate point-of-care reconstitution.

The fifth component (osmolar component) may also be combined with the first, second, or third component as either a solution or dry powder. If all components are dry powder, then an additional premeasured component of liquid diluent (e.g. sterile water) will be provided for immediate point-of-care reconstitution.

Similarly, the sixth component, if present, may also be combined with the first, second, or third component as either a solution or dry powder. If all components are dry powder, then an additional premeasured component of liquid diluent (e.g. sterile water) will be provided for immediate point-of-care reconstitution.

In another aspect of the invention, the multipart kit will contain components of the formulations described above, either as new separate components or combined with pre-existing components. It is obvious to those skilled in the art that components can only be combined together in the kit if they are stable and do not react with each other.

Concentrations and quantities of components of the multipart kit are as described above when the elements comprising the multipart kit are dissolved in water or another aqueous fluid for administration. Also, the quantity of the acute-acting anesthetic lidocaine that is present in a unit dose is as described above. Similarly, the quantity of anionic polysaccharide such as heparin that is present in a unit dose is as described above. Also similarly, the quantity of buffer such as sodium bicarbonate buffer that is present in a unit dose is as described above. If the osmolar component is present, the quantity of the osmolar component such as sodium chloride that is present in a unit dose is as described above.

In a kit according to the present invention, components are typically provided as sodium or calcium salts, if the components are salts, such as heparin sodium or the osmolar component as sodium chloride. Sodium is compatible with tissues and will serve to displace or partially displace bound potassium which is very noxious to tissues causing smooth muscle contractions, and trigger neurons causing the sensations of pain and urgency if access to underlying bladder tissues occurs. Notwithstanding theory, potassium salts of components are counter to the invention due to the noxious nature of potassium on damaged bladder tissues and subsidiary layers.

The kit can be composed of premeasured components in two or more separate vials. Alternatively the premeasured components may be packaged in a prefilled syringe in combination with one or more prefilled vials or a prefilled syringe with two compartments that can be mixed together just prior to bladder instillation. In one embodiment, components are in one compartment and a second compartment of sterile water is provided for point-of-care dissolution. In another embodiment one or more components of the composition are dissolved in the water in one compartment and the remaining components are dry in another compartment and are combined together prior to use. In one embodiment the prefilled syringe has two compartments separated by a nonpermeable membrane in between that is broken just prior to instillation allowing all the components to mix. For ease of use, the kit may also contain a syringe and/or a catheter. It should be obvious to those skilled in the art that only components that are stable in solution or as dry components should be combined with each other in the kit.

In this kit, the compound that enables persistence of the composition to the surface of the bladder epithelium is typically an activatable gelling agent. The activatable gelling agent is as described above. Particularly preferred activatable gelling agents are thermoreversible gelling agents. These include Pluronics F127 gel, Lutrol gel, NASI-containing polymers (N-isopropylacrylamide, ethylmethacrylate, N-acryloxysuccinimide), xyloglucan sols of 1-2%, graft copolymers of pluronic and poly(acrylic acid), pluronic-chitosan hydrogels, and a [Poly(ethylene glycol)-Poly[lactic acid-co-glycolic acid]-Poly(ethylene glycol)) (PEG-PLGA-PEG) polymer.

Similarly, the antibacterial agent, the antifungal agent, and the vasoconstrictor are as described above.

The kit can be composed of premeasured components in two or more separate vials. Alternatively the premeasured components may be packaged in a prefilled syringe in combination with one or more prefilled vials or a prefilled syringe with two compartments that can be mixed together just prior to bladder instillation. In one embodiment, components are in one compartment and a second compartment of sterile water is provided for point-of-care dissolution. In another embodiment one or more components of the composition are dissolved in the water in one compartment and the remaining components are dry in another compartment and are combined together prior to use. In one embodiment the prefilled syringe has two compartments separated by a nonpermeable membrane in between that is broken just prior to instillation allowing all the components to mix. For ease of use, the kit may also contain a syringe and/or a catheter. It should be obvious to those skilled in the art that only components that are stable in solution or as dry components should be combined with each other in the kit.

As used herein, the terms "treat, ameliorate, or prevent" refer to any detectable improvement, whether subjective or objective, in the lower urinary tract disorder of the subject to whom the composition is administered. For example, the terms "treat, ameliorate, or prevent" can refer to an improvement as determined by the PORIS scale, PUF scale, or any component of those scales; reduction of pain; reduction of urinary frequency; reduction of urinary urgency; reduction of requirement for narcotic administration; reduction of incontinence; reduction of abnormal permeability of the urothelium to potassium; or improvement in more than one of these parameters. The terms "treat, ameliorate, or prevent" do not state or imply a cure for the underlying lower urinary tract condition.

In general, when a composition according to the present invention is used in treatment methods, the method comprises the step of administering the composition according to the present invention to a subject with a diagnosed lower urinary tract disorder or with one or more symptoms of pain, urinary urge, urinary frequency, or incontinence in a quantity sufficient to treat or ameliorate the lower urinary tract disorder.

In general, when a multipart kit according to the present invention is used in treatment methods, the method of treating or ameliorating a lower urinary tract disorder comprises the steps of combining the premeasured components of the kit and then administering to a subject with a diagnosed lower urinary tract disorder or with one or more symptoms of pain, urinary urge, urinary frequency, or incontinence a quantity of the composition produced by the step of combining the premeasured components of the kit sufficient to treat or ameliorate the lower urinary tract disorder. The premeasured kit and its components and their composition are as described above.

The invention is illustrated by the following Examples (references). These Examples are included for illustrative purposes and are for reference only.

### EXAMPLE 1

Two studies were undertaken on patients with symptoms of pelvic pain and urgency. Patient symptom severity was determined by the PUF questionnaire, Pelvic Pain, Urgency/Frequency questionnaire. Patients were treated with an intravesical instillation of formulations described below and then therapeutic efficacy was assessed within 30 min to 1 hour by the PORIS questionnaire as shown in Figure 1, Patient Overall Rating of Improvement of Symptoms. Patients were followed up 24 hr after treatment and asked about the duration of benefit, if any, of the treatment.

The composition of the formulations is provided in Table 4 below. The HB-160 mg and the LB-160 mg formulations are provided as part of the current disclosure.

**Table 4**

| **Component** | **HB-160 mg** | **LB-160 mg** | **LB-80 mg** |
|---|---|---|---|
| **Lidocaine HCl** | 46 mM (160 mg) | 37 mM (160 mg) | 18.5 mM (80 mg) |
| **Heparin Sodium** | 3,333 u/ml | 2,666 u/ml | 2,666 u/ml |
| **Sodium Bicarbonate** | 0.33 M | 0.20M | 0.20 M |
| **Sodium Chloride**** | 28.5 mM | 77.5 mM | 77.5 mM |
| **Total Volume** | 12 ml | 15 ml | 15 ml |

| | | | |
|---|---|---|---|
| ** The sodium chloride is provided as a separate component, however, please note that additional sodium ions are also supplied by the sodium salts of bicarbonate and heparin and additional chloride ions are provided by the anion salt of lidocaine and this additional "sodium chloride" is not corrected for in the table. | | | |

In the first study, two groups of patients were treated with two different formulations. One designated here low buffer with 80 mg lidocaine, LB-80 mg, and the other low buffer with 160 mg lidocaine, LB-160 mg that is provided by this invention. After one instillation of LB-80 mg, 35 of 47 patients, 75% experienced significant immediate relief of both pain and urgency as defined by a 50% or greater improvement "moderately improved" on the PORIS scale. In another arm of this study 33 of 35 patients, 94%, experienced significant immediate relief of both pain and urgency as defined by a 50% or greater improvement "moderately improved" on the PORIS scale. A followup phone call was used to monitor duration of effect in patients that had received one instillation of LB-160 mg, and 50% of these patients experienced at least 4 hours of symptom relief.

In the second study, the formulation, HB-160 mg provided by this invention was tested on 26 patients. Of the patients treated with HB-160 mg, most had fairly severe symptoms of pain and/or urge with 15/26 (58%) having PUF scores >20; all patients had PUF scores greater than 15. As shown in Figure 2, 100% of patients experienced significant immediate relief of both pain and urgency as defined by a 50% or greater improvement "moderately improved" on the PORIS scale. Notably, 6 patients were on chronic narcotic use for greater than 6 months, and 4/6 of these patients experienced 100% relief on PORIS scale and the remaining 2/6 experienced 75% relief on PORIS scale. In contrast, the LB-160 mg formulation has only provided limited to no benefit to this severe class of patients experiencing severe chronic pelvic pain, urgency and frequency.

Seemingly, the difference between 94% of patients experiencing "moderate improvement" with the LB-160 mg formulation versus the 100% of patients experiencing "moderate improvement" with the HB-160 mg may seem to be a minor increase in efficacy. However, as shown in Figure 3 the distribution of PORIS scores is significantly shifted to the right with statistically significant P value of 0.009, with nearly 80% of patients treated with HB-160 mg experiencing a 100% improvement in symptoms meaning that their symptoms were gone. In contrast, only a little over 40% of patients treated with LB-160 mg had their "symptoms gone" demonstrating that HB-160 mg had doubled the proportion of patients in the 100% improvement symptom class. Additionally, all patients treated, even those severe interstitial cystitis (IC) patients, experienced some benefit as there were no patients in the "worse", "no better" or "slightly improved" class of symptoms with "slightly improved" equivalent to up to 25% improvement of combined symptoms of pain and urgency.

Another significant difference between HB-160 mg and LB-160 mg was in the duration of relief experienced by patients. Patients were followed up 24-48 hr after their treatment and asked about the duration of relief from symptoms of pain and/or urgency. As previously mentioned, the average duration of relief for patients treated with LB-160 mg was 4 hours, however, the distribution of relief of the patients is graphed in Figure 3 and approximately 50% of patients treated with LB-160 mg only experienced 1-4 hours of relief which is not dramatically longer than the half-life of the anesthetic component lidocaine which has a half-life of 1.5 hr. In contrast, patients treated with HB-160 mg experienced a significant longer average duration of benefit of 7 hours. Only a minority of these patients, approximately 15% experienced 1-4 hours of relief which is very different from the 50% of patients treated with the LB-160 mg.

### EXAMPLE 2

A small study was undertaken on another modification of the formulation designated HB-200 mg. The total lidocaine dose per treatment was increased to 200 mg and the total amount of the other components was altered as shown in the following table 5. A total of 15 patients known to have significant symptoms of pelvic pain and urgency were administered the HB-200 mg solution via an intravesical instillation. All 15 patients experienced a significant reduction in their symptoms of pain and urgency however, one patient did experience a slight headache which is a side-effect of elevated systemic levels of lidocaine. This was not severe and was readily reversible by lowering the lidocaine dosage.

**Table 5**

| **Component** | **HB-200 mg** | **HB-160 mg** | **LB-160 mg** | **LB-80 mg** |
|---|---|---|---|---|
| **Lidocaine HCl** | 53 mM (200 mg) | 46 mM (160 mg) | 37 mM (160 mg) | 18.5 mM (80 mg) |
| **Heparin Sodium** | 3,076 u/ml | 3,333 u/ml | 2,666 u/ml | 2,666 u/ml |
| **Sodium Bicarbonate** | 0.30 M | 0.33 M | 0.20M | 0.20 M |
| **Sodium Chloride**** | 26.3 mM | 28.5 mM | 77.5 mM | 77.5 mM |
| **Total Volume** | 13 ml | 12 ml | 15 ml | 15 ml |

### ADVANTAGES OF THE INVENTION

Kits and improved compositions according to the present invention provide an improved way of treating a number of chronic, hard-to-treat lower urinary tract conditions that affect a large number of people, many of whom have not been diagnosed properly. These kits, and improved compositions are directed towards resolving the pathophysiological basis of the conditions by reducing the abnormal permeability of the bladder epithelium and also desensitizing the activity of the nerves involved in the condition. They can be used together with other therapies for symptoms such as pain, if desired, do not cause significant side effects, and are well tolerated.

Moreover, the use of kits and improved compositions according to the present invention provide rapid relief and do not require extended periods of time, such as several months, to provide relief. This is particularly important and provides a clear advantage over previous treatment methods.

## Claims

1. A pharmaceutical composition that is an intravesical formulation for bladder instillation for treating or ameliorating a lower urinary tract disorder selected from the group consisting of interstitial cystitis and radiation-induced cystitis comprising:
(a) an anionic polysaccharide in a quantity sufficient to treat or ameliorate the lower urinary tract disorder, wherein the anionic polysaccharide is selected from the group consisting of hyaluronic acid, hyaluronan, chondroitin sulfate, pentosan polysulfate, dermatan sulfates, heparin, heparan sulfates, and keratan sulfates;
(b) lidocaine in a quantity sufficient to treat or ameliorate the lower urinary tract disorder;
(c) a buffer that buffers the solution at a pH that ensures that a sufficient portion of the lidocaine is present in an uncharged state so that the lidocaine can cross the cell membranes, wherein the buffer is selected from the group consisting of sodium bicarbonate, phosphate buffer, and Tris buffer, and the buffer is present in a quantity such that the buffer has a buffering capacity at least equivalent to the buffering capacity of a quantity of sodium bicarbonate such that, when the formulation is dissolved in an aqueous liquid for administration, the sodium bicarbonate is present at a concentration of 0.20 M to 0.45 M, and wherein the pH at which the solution is buffered is from 7.3 to 7.7;
(d) optionally, an osmolar component that provides an isotonic or nearly isotonic solution compatible with human cells and blood, wherein the osmolar component is selected from the group consisting of sodium chloride, dextrose, dextran 40, dextran 60, and mannitol; and
(e) optionally, an additional component comprising one or more of the following in any combination:
(i) a compound that enables persistence of the composition to the surface of the bladder epithelium in a quantity sufficient to treat or ameliorate the lower urinary tract disorder, wherein the compound that enables persistence of the composition to the surface of the bladder epithelium is a thermoreversible gelling agent;
(ii) an antibacterial agent in a quantity sufficient to treat or ameliorate the lower urinary tract disorder;
(iii) an antifungal agent in a quantity sufficient to treat or ameliorate the lower urinary tract disorder; and
(iv) a vasoconstrictor agent in a quantity sufficient to treat or ameliorate the lower urinary tract disorder.

2. The pharmaceutical composition of claim 1 wherein the anionic polysaccharide is heparin, and wherein the quantity of heparin in the pharmaceutical composition is from 10,000 units to 100,000 units per unit dose of the composition.

3. The pharmaceutical composition of claim 2 wherein the quantity of heparin in the pharmaceutical composition is from 25,000 units to 60,000 units per unit dose of the composition.

4. The pharmaceutical composition of claim 1 wherein the buffer is sodium bicarbonate buffer.

5. The pharmaceutical composition of claim 1 wherein the buffer is phosphate buffer.

6. The pharmaceutical composition of claim 1 wherein the buffer is Tris buffer.

7. The pharmaceutical composition of claim 1 wherein the quantity of lidocaine in the composition is such that a unit dose contains 160 mg to 240 mg of lidocaine.

8. The pharmaceutical composition of claim 1 wherein the pH at which the solution is buffered is 7.4 to 7.5.

9. A multipart kit of two or more separate premeasured components comprising:
(a) a first component that comprises a pharmaceutical composition comprising a solution or dry powder comprising an anionic polysaccharide in a quantity sufficient to treat or ameliorate a lower urinary tract disorder selected from the group consisting of interstitial cystitis and radiation-induced cystitis, wherein the anionic polysaccharide is selected from the group consisting of hyaluronic acid, hyaluronan, chondroitin sulfate, pentosan polysulfate, dermatan sulfates, heparin, heparan sulfates, and keratan sulfates;
(b) a second component that comprises lidocaine as a solution or dry powder in a quantity sufficient to treat or ameliorate a lower urinary tract disorder; and
(c) a third component that comprises a buffer either as a solution or as a dry powder that buffers the solution at a pH that ensures that a sufficient portion of the lidocaine is present in an uncharged state so that the lidocaine can cross the cell membranes, wherein the third component can optionally be combined with the second component as either a solution or dry powder, and wherein the buffer is selected from the group consisting of sodium bicarbonate, phosphate buffer, and Tris buffer, and the buffer is present in a quantity such that the buffer has a buffering capacity at least equivalent to the buffering capacity of a quantity of sodium bicarbonate such that, when the formulation is dissolved in an aqueous liquid for administration, the sodium bicarbonate is present at a concentration of 0.20 M to 0.45 M and wherein the pH at which the solution is buffered is from 7.3 to 7.7;
(d) optionally, when the first, second, and third components are all dry powder, a fourth component that comprises a premeasured component of liquid diluent;
(e) optionally, a fifth premeasured component comprising an osmolar component wherein the osmolar component is selected from the group consisting of sodium chloride, dextrose, dextran 40, dextran 60, and mannitol; wherein the fifth premeasured component can optionally be combined with any of the other components if stable; and
(f) optionally, a sixth premeasured component comprising one or more of the following in any combination:
(i) a compound that enables persistence of the composition to the surface of the bladder epithelium, wherein the compound that enables persistence of the composition to the surface of the bladder epithelium is a thermoreversible gelling agent;
(ii) an antibacterial agent;
(iii) an antifungal agent; and
(iv) a vasoconstrictor; wherein the sixth premeasured component can optionally be combined with any of the other components if stable;
wherein the multipart kit is combined to produce a composition that is an intravesical formulation for bladder instillation.

10. The multipart kit of claim 9 wherein the anionic polysaccharide is heparin, and wherein the quantity of heparin in the multipart kit is from 10,000 units to 100,000 units per unit dose.

11. The multipart kit of claim 10 wherein the quantity of heparin in the multipart kit is from 25,000 units to 60,000 units per unit dose.

12. The multipart kit of claim 9 wherein the buffer is sodium bicarbonate buffer.

13. The multipart kit of claim 9 wherein the buffer is phosphate buffer.

14. The multipart kit of claim 9 wherein the buffer is Tris buffer.

15. The multipart kit of claim 9 wherein the quantity of lidocaine in the multipart kit is such that a unit dose contains 160 mg to 240 mg of lidocaine.

16. The multipart kit of claim 9 wherein the kit is packaged in two or more compartments.

17. The multipart kit of claim 16 wherein the compartments are two or more vials, one or more vials and a prefilled syringe, or a prefilled syringe with two compartments and a nonpermeable membrane/stopper in between that is broken/moved just prior to instillation allowing all the components to mix.

18. The multipart kit of claim 9 wherein the pH at which the solution is buffered is from 7.4 to 7.5.

## Patentansprüche

1. Pharmazeutische Zusammensetzung, die eine intravesikale Formulierung für die Blaseninstillation zur Behandlung oder Verbesserung einer Erkrankung der unteren Harnwege ist, ausgewählt aus der Gruppe bestehend aus interstitieller Blasenentzündung und strahlungsinduzierter Blasenentzündung, umfassend:
(a) ein anionisches Polysaccharid in einer ausreichenden Menge, um die Erkrankung der unteren Harnwege zu behandeln oder zu verbessern, wobei das anionische Polysaccharid ausgewählt ist aus der Gruppe bestehend aus Hyaluronsäure, Hyaluronsäure, Hyaluronan, Chondroitinsulfat, Pentosanpolysulfat, Dermatansulfaten, Heparin, Heparansulfaten und Keratansulfaten;
b) Lidocain in einer ausreichenden Menge, um die Erkrankung der unteren Harnwege zu behandeln oder zu verbessern;
(c) einen Puffer, der die Lösung bei einem pH-Wert puffert, der sicherstellt, dass ein ausreichender Teil des Lidocains in einem ungeladenen Zustand vorliegt, so dass das Lidocain die Zellmembranen durchqueren kann, wobei der Puffer ausgewählt ist aus der Gruppe bestehend aus Natriumhydrogencarbonat, Phosphatpuffer und Tris-Puffer, und der Puffer in einer solchen Menge vorhanden ist, dass der Puffer eine Pufferkapazität aufweist, die mindestens der Pufferkapazität einer Menge Natriumbikarbonat entspricht, so dass, wenn die Formulierung in einer wässrigen Flüssigkeit zur Verabreichung gelöst ist, das Natriumbikarbonat in einer Konzentration von 0,20 M bis 0,45 M vorhanden ist, und wobei der pH-Wert, bei dem die Lösung gepuffert wird, 7,3 bis 7,7 beträgt;
(d) gegebenenfalls eine osmolare Komponente, die eine isotonische oder nahezu isotonische Lösung bereitstellt, die mit menschlichen Zellen und Blut kompatibel ist, wobei die osmolare Komponente ausgewählt ist aus der Gruppe bestehend aus Natriumchlorid, Dextrose, Dextran 40, Dextran 60 und Mannitol; und
(e) gegebenenfalls eine zusätzliche Komponente, die eine oder mehrere der folgenden Komponenten in beliebiger Kombination umfasst:
(i) eine Verbindung, die das Anhalten der Zusammensetzung an der Oberfläche des Blasenepithels in einer ausreichenden Menge ermöglicht, um die Erkrankung der unteren Harnwege zu behandeln oder zu verbessern, wobei die Verbindung, die das Anhalten der Zusammensetzung an der Oberfläche des Blasenepithels ermöglicht, ein thermoreversibles Geliermittel ist;
(ii) ein antibakterielles Mittel in einer ausreichenden Menge, um die Erkrankung der unteren Harnwege zu behandeln oder zu verbessern;
(iii) ein Antipilzmittel in einer ausreichenden Menge, um die Erkrankung der unteren Harnwege zu behandeln oder zu verbessern; und
(iv) ein vasokonstriktorisches Mittel in einer ausreichenden Menge, um die Erkrankung der unteren Harnwege zu behandeln oder zu verbessern.

2. Pharmazeutische Zusammensetzung nach Anspruch 1, wobei das anionische Polysaccharid Heparin ist, und wobei die Menge an Heparin in der pharmazeutischen Zusammensetzung von 10.000 Einheiten bis 100.000 Einheiten pro Einzeldosis der Zusammensetzung beträgt.

3. Pharmazeutische Zusammensetzung nach Anspruch 2, wobei die Menge an Heparin in der pharmazeutischen Zusammensetzung von 25.000 Einheiten bis 60.000 Einheiten pro Einzeldosis der Zusammensetzung beträgt.

4. Pharmazeutische Zusammensetzung nach Anspruch 1, wobei der Puffer Natriumbikarbonatpuffer ist.

5. Pharmazeutische Zusammensetzung nach Anspruch 1, wobei der Puffer Phosphatpuffer ist.

6. Pharmazeutische Zusammensetzung nach Anspruch 1, wobei der Puffer Tris-Puffer ist.

7. Pharmazeutische Zusammensetzung nach Anspruch 1, wobei die Menge an Lidocain in der Zusammensetzung so ist, dass eine Einzeldosis 160 mg bis 240 mg Lidocain enthält.

8. Pharmazeutische Zusammensetzung nach Anspruch 1, wobei der pH-Wert, bei dem die Lösung gepuffert wird, 7,4 bis 7,5 beträgt.

9. Mehrteiliger Kit aus zwei oder mehr separaten, vorgemessenen Komponenten, umfassend:
(a) eine erste Komponente, die eine pharmazeutische Zusammensetzung umfasst, die eine Lösung oder ein Trockenpulver umfasst, die ein anionisches Polysaccharid in einer ausreichenden Menge umfasst, um eine Erkrankung der unteren Harnwege zu behandeln oder zu verbessern, die aus der Gruppe ausgewählt ist, die aus interstitieller Blasenentzündung und strahlungsinduzierter Blasenentzündung besteht, wobei das anionische Polysaccharid ausgewählt ist aus der Gruppe bestehend aus Hyaluronsäure, Hyaluronan, Chondroitinsulfat, Pentosanpolysulfat, Dermatansulfaten, Heparin, Heparansulfaten und Keratansulfaten;
(b) eine zweite Komponente, die Lidocain als Lösung oder Trockenpulver in einer ausreichenden Menge umfasst, um eine Erkrankung der unteren Harnwege zu behandeln oder zu verbessern; und
(c) eine dritte Komponente, die einen Puffer entweder als Lösung oder als Trockenpulver umfasst, der die Lösung bei einem pH-Wert puffert, der sicherstellt, dass ein ausreichender Teil des Lidocains in einem ungeladenen Zustand vorliegt, so dass das Lidocain die Zellmembranen durchqueren kann, wobei die dritte Komponente optional mit der zweiten Komponente entweder als Lösung oder als Trockenpulver kombiniert werden kann, und wobei der Puffer ausgewählt ist aus der Gruppe bestehend aus Natriumbikarbonat, Phosphatpuffer und Tris-Puffer, und der Puffer in einer solchen Menge vorhanden ist, dass der Puffer eine Pufferkapazität aufweist, die mindestens der Pufferkapazität einer Menge Natriumbikarbonat entspricht, so dass, wenn die Formulierung in einer wässrigen Flüssigkeit zur Verabreichung gelöst ist, das Natriumbikarbonat in einer Konzentration von 0,20 M bis 0,45 M vorhanden ist und wobei der pH-Wert, bei dem die Lösung gepuffert wird, 7,3 bis 7,7 beträgt;
(d) gegebenenfalls, wenn die erste, zweite und dritte Komponente alle Trockenpulver sind, eine vierte Komponente, die eine vorgemessene Komponente des flüssigen Verdünnungsmittels umfasst;
(e) gegebenenfalls eine fünfte vorgemessene Komponente, die eine osmolare Komponente umfasst, wobei die osmolare Komponente ausgewählt ist aus der Gruppe bestehend aus Natriumchlorid, Dextrose, Dextran 40, Dextran 60 und Mannitol; wobei die fünfte vorgemessene Komponente gegebenenfalls mit jeder der anderen Komponenten kombiniert werden kann, wenn sie stabil ist; und
(f) gegebenenfalls eine sechste vorgemessene Komponente, die eine oder mehrere der folgenden Komponenten in beliebiger Kombination umfasst:
(i) eine Verbindung, die das Anhalten der Zusammensetzung an der Oberfläche des Blasenepithels ermöglicht, wobei die Verbindung, die das Anhalten der Zusammensetzung an der Oberfläche des Blasenepithels ermöglicht, ein thermoreversibles Geliermittel ist;
(ii) ein antibakterielles Mittel;
(iii) ein Antipilzmittel; und
(iv) vasokonstriktorisches Mittel; wobei die sechste vorgemessene Komponente gegebenenfalls mit jeder der anderen Komponenten kombiniert werden kann, wenn sie stabil ist;
wobei der mehrteilige Kit kombiniert wird, um eine Zusammensetzung herzustellen, die eine intravesikale Formulierung für die Blaseninstillation ist.

10. Mehrteiliger Kit nach Anspruch 9, wobei das anionische Polysaccharid Heparin ist, und wobei die Menge an Heparin in dem mehrteiligen Kit von 10.000 Einheiten bis 100.000 Einheiten pro Einzeldosis beträgt.

11. Mehrteiliger Kit nach Anspruch 10, wobei die Menge an Heparin in dem mehrteiligen Kit von 25.000 Einheiten bis 60.000 Einheiten pro Einzeldosis beträgt.

12. Mehrteiliger Kit nach Anspruch 9, wobei der Puffer Natriumbikarbonatpuffer ist.

13. Mehrteiliger Kit nach Anspruch 9, wobei der Puffer Phosphatpuffer ist.

14. Mehrteiliger Satz nach Anspruch 9, wobei der Puffer Tris-Puffer ist.

15. Mehrteiliger Kit nach Anspruch 9, wobei die Menge an Lidocain in dem mehrteiligen Kit so ist, dass eine Einzeldosis 160 mg bis 240 mg Lidocain enthält.

16. Mehrteiliger Satz nach Anspruch 9, wobei der Kit in zwei oder mehr Abteile verpackt ist.

17. Mehrteiliger Kit nach Anspruch 16, wobei die Abteile zwei oder mehr Fläschchen, ein oder mehrere Fläschchen und eine vorgefüllte Spritze sind, oder eine vorgefüllte Spritze mit zwei Abteile und einer undurchlässigen Membran/Stopper dazwischen, die kurz vor der Instillation gebrochen/bewegt wird, so dass sich alle Komponenten vermischen können.

18. Mehrteiliger Kit nach Anspruch 9, wobei der pH-Wert, bei dem die Lösung gepuffert wird, 7,4 bis 7,5 beträgt.

## Revendications

1. Composition pharmaceutique qui est une formulation intravésicale pour une instillation de la vessie afin de traiter ou améliorer un trouble des voies urinaires inférieures choisi parmi le groupe comprenant une cystite interstitielle et une cystite induite par rayonnement, comprenant :
(a) un polysaccharide anionique en quantité suffisante pour traiter ou améliorer le trouble des voies urinaires inférieures, le polysaccharide anionique étant choisi parmi le groupe constitué de l'acide hyaluronique, hyaluronane, sulfate de chondroïtine, polysulfate de pentosan, sulfates de dermatane, héparine, sulfates d'héparane, et sulfates de kératane ;
(b) de la lidocaïne en quantité suffisante pour traiter ou améliorer le trouble des voies urinaires inférieures ;
(c) un tampon qui tamponne la solution à un pH garantissant qu'une partie suffisante de la lidocaïne est présente dans un état non chargé, de sorte que la lidocaïne peut traverser les membranes cellulaires, le tampon étant choisi parmi le groupe constitué de bicarbonate de sodium, tampon phosphate, et tampon Tris, et le tampon est présent en quantité telle que le tampon a une capacité tampon au moins équivalente à la capacité tampon d'une quantité de bicarbonate de sodium telle que, lorsque la formulation est dissoute dans un liquide aqueux pour administration, le bicarbonate de sodium est présent à une concentration entre 0,20 M et 0,45 M, et le pH auquel la solution est tamponnée est de 7,3 à 7,7 ;
(d) facultativement, un composant osmolaire qui fournit une solution isotonique ou pratiquement isotonique compatible avec des cellules et du sang humains, le composant osmolaire étant choisi parmi le groupe constitué de chlorure de sodium, dextrose, dextran 40, dextran 60, et mannitol ; et
(e) facultativement, un composant supplémentaire comprenant un ou plusieurs des éléments suivants selon une combinaison quelconque :
(i) un composé qui permet une persistance de la composition à la surface de l'épithélium de vessie en quantité suffisante pour traiter ou améliorer le trouble des voies urinaires inférieures, le composé qui permet une persistance de la composition à la surface de l'épithélium de vessie étant un agent gélifiant thermoréversible ;
(ii) un agent antibactérien en quantité suffisante pour traiter ou améliorer le trouble des voies urinaires inférieures ;
(iii) un agent antifongique en quantité suffisante pour traiter ou améliorer le trouble des voies urinaires inférieures ; et
(iv) un agent vasoconstricteur en quantité suffisante pour traiter ou améliorer le trouble des voies urinaires inférieures.

2. Composition pharmaceutique selon la revendication 1, dans laquelle le polysaccharide anionique est de l'héparine, et dans laquelle la quantité d'héparine dans la composition pharmaceutique est comprise entre 10 000 et 100 000 unités par dose unitaire de la composition.

3. Composition pharmaceutique selon la revendication 2, dans laquelle la quantité d'héparine dans la composition pharmaceutique est comprise entre 25 000 et 60 000 unités par dose unitaire de la composition.

4. Composition pharmaceutique selon la revendication 1, dans laquelle le tampon est un tampon de bicarbonate de sodium.

5. Composition pharmaceutique selon la revendication 1, dans laquelle le tampon est un tampon de phosphate.

6. Composition pharmaceutique selon la revendication 1, dans laquelle le tampon est un tampon Tris.

7. Composition pharmaceutique selon la revendication 1, dans laquelle la quantité de lidocaïne dans la composition est telle qu'une dose unitaire contient de 160 mg à 240 mg de lidocaïne.

8. Composition pharmaceutique selon la revendication 1, dans laquelle le pH auquel la solution est tamponnée est de 7,4 à 7,5.

9. Kit en plusieurs parties constitué de deux ou plus de deux composants séparés préalablement mesurés comprenant :
(a) un premier composant qui comprend une composition pharmaceutique comprenant une solution ou une poudre sèche comprenant un polysaccharide anionique en quantité suffisante pour traiter ou améliorer un trouble des voies urinaires inférieures choisi parmi le groupe constitué d'une cystite interstitielle et d'une cystite induite par rayonnement, dans lequel le polysaccharide anionique est choisi parmi le groupe constitué de l'acide hyaluronique, hyaluronane, sulfate de chondroïtine, polysulfate de pentosan, sulfates de dermatane, héparine, sulfates d'héparane, et sulfates de kératane ;
(b) un deuxième composant qui comprend de la lidocaïne sous forme de solution ou de poudre sèche en quantité suffisante pour traiter ou améliorer un trouble des voies urinaires inférieures ; et
(c) un troisième composant qui comprend un tampon sous forme d'une solution ou d'une poudre sèche qui tamponne la solution à un pH garantissant qu'une partie suffisante de la lidocaïne est présente dans un état non chargé, de sorte que la lidocaïne peut traverser les membranes cellulaires, dans lequel le troisième composant peut facultativement être combiné avec le deuxième composant sous forme d'une solution ou d'une poudre sèche, le tampon étant choisi parmi le groupe constitué de bicarbonate de sodium, tampon phosphate, et tampon Tris, et le tampon est présent en quantité telle que le tampon a une capacité tampon au moins équivalente à la capacité tampon d'une quantité de bicarbonate de sodium telle que, lorsque la formulation est dissoute dans un liquide aqueux pour administration, le bicarbonate de sodium est présent à une concentration entre 0,20 M et 0,45 M, et le pH auquel la solution est tamponnée est de 7,3 à 7,7 ;
(d) facultativement, lorsque les premier, deuxième et troisième composants sont tous en poudre sèche, un quatrième composant qui comprend un composant de diluant liquide préalablement mesuré ;
(e) facultativement, un cinquième composant préalablement mesuré comprenant un composant osmolaire dans lequel le composant osmolaire est choisi parmi le groupe constitué de chlorure de sodium, dextrose, dextran 40, dextran 60, et mannitol ; dans lequel le cinquième composant préalablement mesuré peut facultativement être combiné avec l'un quelconque des autres composants s'il est stable ; et
(f) facultativement, un sixième composant préalablement mesuré comprenant un ou plusieurs des éléments suivants selon une combinaison quelconque :
(i) un composé qui permet une persistance de la composition à la surface de l'épithélium de vessie, le composé qui permet une persistance de la composition à la surface de l'épithélium de vessie étant un agent gélifiant thermoréversible ;
(ii) un agent antibactérien ;
(iii) un agent antifongique ; et
(iv) un vasoconstricteur ; dans lequel le sixième composant préalablement mesuré peut facultativement être combiné avec l'un quelconque des autres composants s'il est stable ;
dans lequel le kit en plusieurs parties est combiné pour produire une composition qui est une formulation intravésicale pour une instillation de la vessie.

10. Kit en plusieurs parties selon la revendication 9, dans lequel le polysaccharide anionique est de l'héparine, et dans lequel la quantité d'héparine dans le kit en plusieurs parties va de 10 000 à 100 000 unités par dose unitaire.

11. Kit en plusieurs parties selon la revendication 10, dans lequel la quantité d'héparine dans le kit en plusieurs parties va de 25 000 unités à 60 000 unités par dose unitaire.

12. Kit en plusieurs parties selon la revendication 9, dans lequel le tampon est un tampon de bicarbonate de sodium.

13. Kit en plusieurs parties selon la revendication 9, dans lequel le tampon est un tampon de phosphate.

14. Kit en plusieurs parties selon la revendication 9, dans lequel le tampon est un tampon Tris.

15. Kit en plusieurs parties selon la revendication 9, dans lequel la quantité de lidocaïne dans le kit en plusieurs parties est telle qu'une dose unitaire contient de 160 mg à 240 mg de lidocaïne.

16. Kit en plusieurs parties selon la revendication 9, dans lequel le kit est emballé dans deux ou plus de deux compartiments.

17. Kit en plusieurs parties selon la revendication 16, dans lequel les compartiments sont deux ou plus de deux flacons, un ou plusieurs flacons et une seringue préremplie, ou une seringue préremplie avec deux compartiments et une membrane/un bouchon non perméable entre ceux-ci qui est cassé/déplacé juste avant l'instillation, permettant à tous les composants de se mélanger.

18. Kit en plusieurs parties selon la revendication 9, dans lequel le pH auquel la solution est tamponnée est compris entre 7,4 et 7,5.
